# EUROPEAN PATENT APPLICATION

(11) **EP 2 314 646 A1**
(43) Date of publication of application: **27.04.2011**
(21) Application number: 09013216.8
(22) Date of filing: 20.10.2009
(51) Int. Cl.: C08L 89/00, C07K 1/113, C07K 5/02, C12N 9/22, C12N 9/36

(54) **Diselenide-resins for oxidative protein folding**

(71) Applicant: ETH Zurich, 8092 Zürich (CH)
(72) Inventor: Hilvert, Donald, 8006 Zürich (CH); Beld, Joris, 8037 Zürich (CH); Woycechowsky, Kenneth J., 84102 Salt Lake City (US)

(57) **Abstract**

A resin comprising a water-compatible polymer and a diselenide compound wherein the diselenide compound is either directly or by a linker covalently bound to the polymer is described. A method for the production of said resins and their use for oxidative protein folding are also provided.

## Description

The present invention relates to resins comprising a diselenide compound which are useful for oxidative protein folding and a method for producing said resins.

Many polypeptides and proteins that are of considerable interest for medical or industrial applications contain disulfide bridges crucial for their three-dimensional structure. And although the recombinant expression of polypeptides and proteins has become a widely used standard method, the production of functional polypeptides and proteins remains often a difficult task, since polypeptides and proteins produced in micro-organisms do not readily acquire their native three-dimensional structure that is compulsory for their proper activity.

The over-production of proteins in micro-organisms often leads to, so called, inclusion bodies, which primarily consist of pure but un- or misfolded protein. Correctly folded protein can be obtained by denaturation and refolding. Disulfide bond containing proteins require oxidative protein folding, often a very slow process requiring redox additives and catalysts. Typically, high concentrations of oxidized and reduced glutathione are used, but recently it was shown that oxidative protein folding can be catalyzed by small molecular diselenides. Nevertheless, these reagents have to be removed from the folded protein, e.g. by chromatography, thereby requiring additional and often laborious steps until the functional, folded protein is obtained. Attaching the protein folding reagent to a solid support facilitates practical oxidative protein folding. However, only a few attempts have been made to create oxidative protein folding resins.

In 1996, Clark and Pai synthesized a range of thiol oxidizing resins,¹ from which a resin containing Ellman's reagent (5,5'-dithiobis-(2-nitrobenzoic acid), DTNB)², called Ekathiox, was commercially available, and used for various cysteine oxidations in peptides and proteins. ^{3,4} A few years later a similar disulfide-bond containing reagent (also a DTNB analog) attached to a newly developed water soluble resin (CLEAR®) was synthesized.⁵ This resin, which is nowadays commercially available as CLEAR-OX®, was used to successfully oxidize conotoxins, showing a clear advantage over solution conditions at high protein concentrations. ^{6,7} Although both DTNB-based resins, Ekathiox and CLEAR-OX, are clearly good protein-thiol oxidants, they have, somewhat surprisingly, not been used for oxidative protein folding, which consists of oxidation and isomerization processes.

Shimizu et al. attached the small molecule dithiotreitol (DTT)⁸ to polymeric microspheres. ^{9,10} Both scrambled as well as fully reduced ribonuclease A could be folded by these functionalized microspheres. Refolding of lysozyme by cystamine modified microspheres showed that the microspheres prevent protein aggregation and increase the folding yields.¹⁰ In a further attempt, Woycechowsky et al. attached a dithiol to resins and microspheres to catalyze the isomerization of scrambled ribonuclease A.¹¹ The microsphere bound dithiol gave a 17-fold increase in yield, whereas attaching the same dithiol to Tentagel®¹² gave only a 2-fold increase in reactivation, suggesting an influence of the composition of polymeric resin used. Recently, three water and organic solvent compatible resins, entirely consisting of polyethylene glycol, have been developed: SPOCC®¹³, CLEAR®¹⁴, and Chemmatrix®. ¹⁵ The three resins have been used in either native chemical ligation, folding and assaying-on-resin,¹⁶ the oxidation of conotoxins, ^{6,7} or the solid phase peptide synthesis of difficult peptide sequences. ¹⁷ All three resins show superior properties compared to traditional resins (Wang, Tentagel, Pega) ¹⁸ regarding swelling in organic solvents and water, and are therefore ideally suited for biochemical experiments.

It has been suggested that, apart from the practical benefits, resin-bound oxidative protein folding reagents offer the advantage of chaperone-like effects, presumably originating from the polymer¹⁰. Shimizu et al.^{9,10} suggested that disulfide-carrying microspheres show a similar behavior as the GroEL/GroES chaperone complex. The reduced protein adsorbs to the polymer, proteinogenic cysteines are oxidized and isomerized and the folded protein has lower affinity for the surface and dissociates. As a consequence, during the oxidation process it is protected from non-specific aggregation. However, whereas natural chaperones²¹ like GroEL/GroES stabilize unfolded or partially folded proteins temporarily, a polymer is a static macromolecule. It is therefore an open question as to how a polymer mimics this chaperone complex. Other chaperones like PDI²² may be a better model for oxidative protein folding by functionalized polymers because PDI has low substrate specificity, contains catalytic disulfide bonds, and has a relative hydrophobic surface for binding unfolded proteins.

A possible chaperone-like effect of non-functionalized resins could originate from macromolecule crowding. In the case of lysozyme refolding, the effect of macromolecular crowding has been investigated. In the presence of crowding reagents (BSA, dextran, Ficoll 70) the folding yields decrease due to increased aggregation, whereas the chaperone PDI prevents it^{23, 24, 25}. However, when folded in 2 M urea, molecular crowding reagents (especially BSA) increased the rate of folding²⁶. Further, it has been shown that low concentrations of BSA alone are able to accelerate the oxidative folding of the two-disulfide containing 13-mer conotoxin GI²⁷

Although, there have been a number of attempts to provide methods for reliable and fast oxidative protein folding, no method available has achieved fast, reliable and efficient oxidative protein folding. This holds particularly true for larger scale industrial processes.

Thus, there is still a great need for agents that provide for a fast and efficient oxidative folding of polypeptides and proteins on a larger industrial scale in a simple and environmental friendly manner.

The problem of the present invention is to provide an agent that achieves a fast and efficient oxidative protein folding in a simple environmental friendly manner on laboratory up to larger scale industrial processes.

The problem is solved by a resin according to claim 1. Further preferred embodiments are the subject-matter of the dependent claims. A method for the production of these resins according to claim 9 is also provided.

"Cross-linked" as used herein refers compounds that are linked to each other by covalent bonds. For example, polymers may be cross-linked. Cross-linked polymers form polymeric networks, hydrogels or resins.

"Water-compatible" as generally used herein means that the resin and the polymer comprised therein are hydrophilic in order to be either water-soluble or at least readily wettable in aqueous solutions. Further, the resin and the polymer comprised therein are mechanically and chemically stable in aqueous buffer solutions.

"Functionalize" as generally used herein refers to modifying a molecule in a manner that results in the attachment of a functional group or moiety. For example, a molecule may be functionalized by the introduction of an amino group. Resins may be functionalized.

A resin according to the present invention comprises a water-compatible polymer and a diselenide compound. The diselenide compound is either directly or by a linker covalently bound to the polymer.

In a preferred embodiment of the resin the water-compatible polymer is selected from the group consisting of poly(alkylene oxide) (PEO), poly (ethylene glycol) (PEG), proteins, polysaccharides, poly(vinyl alcohol) (PVA), polyacrylat (PA), polymethyloxazoline (PMOXA), polyethyloxazoline (PEOXA), polyvinylpyrrolidone (PVP), polylysine, polyarginine, polytyrosine, polyglutamine, polyglutamic acid, polyasparigine, polyaspartic acid, polyserine, polythreonine and polyhistidine, polyallylamine, polyethyleneimine, and any mixture thereof.

The suitable polyaminoacids have a hydrophilic side chain e.g. polylysine, polyarginine, polytyrosine, polyglutamine, polyglutamic acid, polyasparigine, polyaspartic acid, polyserine, polythreonine and polyhistidine. These polymers are hydrophilic, water-soluble or at least readily wettable in or with water.

In a further preferred embodiment the diselenide compound is bound to the polymer by a linker. The linker may comprise more than one part or compound. That is, the linker may be formed by subsequent coupling of two compounds, for example, 4-hydroxymethyl benzoic acid and dibromopropionyl chloride, to the resin.

Often, it is not possible that a diselenide compound is directly bound to the polymer, e.g. due to sterical reasons. Therefore, the diselenide compound may be bound to the polymer by a linker as further defined below.

As used herein, the term "linker" is intended to mean alkyl chains with 1 to 20 carbon atoms, which may be straight or branched and saturated or unsaturated. Examples are methylene, ethylene, n-propylene, i-propylene, n-butylene, i-butylene, sec-butylene, t-butylene, pentylene, hexylene, heptylene, octylene, nonylene, decylene, undecylene, dodecylene, tridecylene, ethenylene, 1-propenylene, 2-propenylene, 1-butenylene, 2-butenylene, 3-butenylene, 3-methyl-2-butenylene, 4-hydroxymethyl benzoic acid and the like. Preferred are methylene, ethylene, n-propylene, i-propylene, n-butylene and dimethylbenzene, and 4-hydroxymethyl benzoic acid. Most preferred is 4-hydroxymethyl benzoic acid. The linker may also comprise one or more heteroatom in the chain or a carbocycle or heterocycle. The heteroatom may be an ether or thioether linkage. Heteroatoms in the chain may also be part of ester, amide or urethane linkage.

Said linker is preferably selected from the group consisting of alkyl chains with 1 to 20 carbon atoms, which may be straight or branched and saturated or unsaturated, dimethylbenzene, and 4-hydroxymethyl benzoic acid. In a more preferred embodiment the linker is 4-hydroxymethyl benzoic acid.

In another preferred embodiment the diselenide compound has a molecular mass of less than 5000Da, preferably less than 2500Da, more preferably of less than 1000Da, even more preferably less than 500Da and most preferably of less than 300Da.

According to a further preferred embodiment the diselenide compound is selected from the group consisting of either straight chain or branched alkyl diselenide compounds, aromatic diselenide compounds. The molecular mass of said diselenide compounds is also less than 5000Da, preferably less than 2500Da, more preferably of less than 1000Da, even more preferably less than 500Da and most preferably of less than 300Da.

The diselenide compound may also comprise a hetero-alkyl chain. "Heteroalkyl" as used herein means an alkyl chain wherein at least one carbon atom of the otherwise alkyl backbone is replaced with a heteroatom, for example, O, S or N. Further, the diselenide compound may also comprise aromatic or heteroaromatic cycles.

In a further preferred embodiment the diselenide compound is selected from the group consisting of selenocystine, selenogluthatione, selenocystamine, bis(2-amino)diphenyldiselenide and 5,5'-diselenobis-(2-nitrobenzoic acid).

A method for the production of a diselenide-resin according to the present invention comprises the following steps. In a first step an optional linker is coupled to the resin. After coupling of said optional linker the diselenide compound is coupled to the linker. In the case that no linker is present, the diselenide compound is directly coupled to the resin. In a further step the diselenide resin is treated with a reducing reagent and subsequently treated with an oxidizing reagent.

In a preferred embodiment of the method the resin is a polyethylene glycol based resin that is functionalized with aminomethyl groups.

In another preferred embodiment the method comprises the steps of coupling of 4-hydroxymethyl benzoic acid to the resin, followed by subsequent coupling of dibromopropionyl chloride to the previously coupled 4-hydroxymethyl benzoic acid. These two compounds form the linker for the diselenide compound to be coupled. In the next step selenocystamine is coupled to the resin via the linker. In the next step the diselenide-resin is treated with the reducing reagent dithiothreitol and subsequently, the diselenide-resin is treated with the oxidizing reagent K₃Fe(CN)₆.

In a further embodiment diselenide-resins according to the present invention are used for oxidative protein folding. Said diselenide-resins can be easily separated from solutions either by centrifugation or filtration which renders them particularly suitable for larger scale production processes of peptides or proteins with disulfide-bonds. The method of production may be a continuous or a batch process.

It is shown a way to attach diselenide reagents to water compatible solid supports. The diselenide-modified polymeric resins can be very easily recycled and show excellent speed and efficiency at folding proteins, even at high protein concentration.

Small molecular diselenides are efficient oxidative protein folding catalysts.²⁸ In order to make the process of preparative oxidative protein folding more practical, the following resins were synthesized here glutathione, selenoglutathione, cystamine and selenocystamine derivatives on CLEAR and Chemmatrix resins. These diselenide functionalized polymers are very practical, recyclable and efficient aids in the oxidative folding of ribonuclease A and lysozyme.

The diselenide resin according to the present invention will be explained in more detail in the following text with reference to exemplary embodiments, which are illustrated in the drawings and in which:
- Fig. 1: shows solid phase peptide synthesis of glutathione (X=S) and selenoglutathione (X=Se) on Chemmatrix and Clear resin;
- Fig. 2: shows solid phase synthesis of resin bound disulfide (X=S) and diselenide (X=Se) reagents;
- Fig. 3: shows oxidative folding of 5 µM RNase A by 1 mg of functionalized resins in pH 8.0 buffer;
- Fig. 4: shows oxidative folding of RNase A at different pH values;
- Fig. 5: shows recycling of resin;
- Fig. 6: shows oxidative folding of lysozyme monitored by an activity assay; and
- Fig. 7: shows the effect of water-compatible CLEAR® and Chemmatrix® resin.

The following nomenclature was adopted for functionalized resins, C+U and C+C are CLEAR® resins functionalized with selenocystamine or cystamine, respectively. M+U and M+C are Chemmatrix® resins functionalized with selenocystamine or cystamine, respectively.

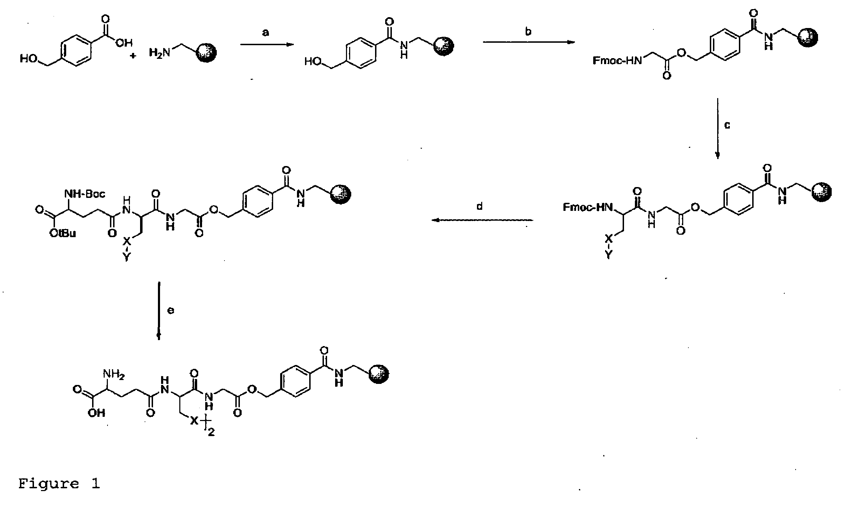

Figure 1 shows solid phase peptide synthesis of glutathione (X=S) and selenoglutathione (X=Se) on Chemmatrix and Clear resin. a) 4-hydroxymethylbenzoic acid (HMBA), HOBt, DIPCDI, b) Fmoc-Gly-OH, MSNT, DIPEA, NMI, c) 1. 20% 4-methylpiperidine, 2. Fmoc-Sec(Mob)-OPfp, HOBt, d) 1. 20% 4-methylpiperidine, 2. Boc-Glu(α-OtBu)OPfp, HOBt, e) TFA, TIPS, 2,2'-(dithiobis)nitropyridine. For glutathione Y=Trt and for selenoglutathione Y=Mob.

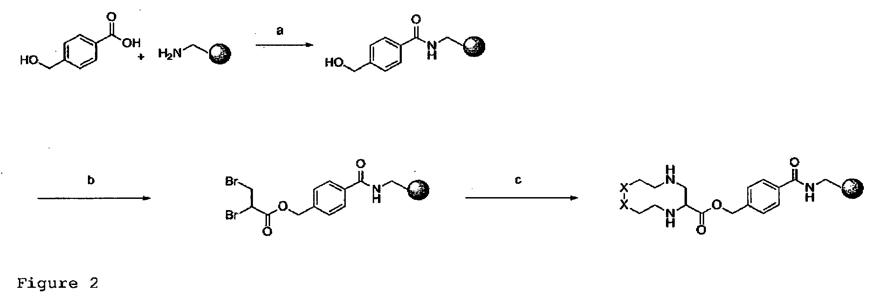

Figure 2 shows solid phase synthesis of resin bound disulfide (X=S) and diselenide (X=Se) reagents. (a) Loading of 4-hydroxymethylbenzoic acid on aminomethyl Chemmatrix or CLEAR-base resin by DIPCDI/HOBt in DMF, (b) attachment of 2,3-dibromopropionyl chloride in the presence of DIPEA in DMF, (c) addition of selenocystamine in the presence of DIPEA in DMF, followed by reduction with DTT and oxidation by K₃Fe(CN)₆;

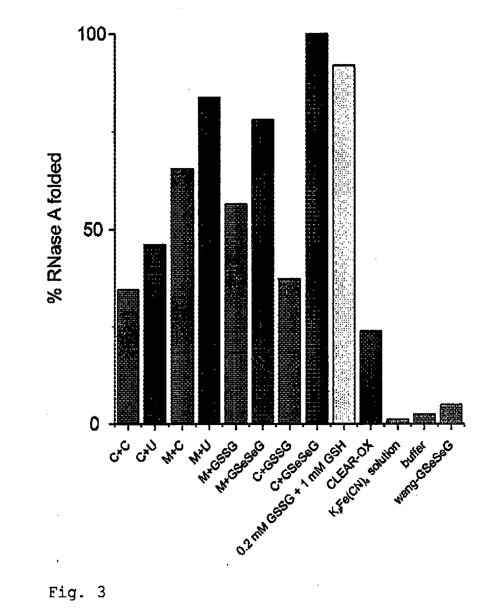

Figure 3 shows oxidative folding of 5 µM RNase A by 1 mg of functionalized resins in pH 8.0 buffer, assayed for RNase A activity after 15h at room temperature. As control experiments, RNase A (5 µM) was also folded in the presence of 1 mg CLEAR-OX, 0.5 M K₃Fe(CN)₆, only buffer and 1 mg GSeSeG functionalized Wang resin.;

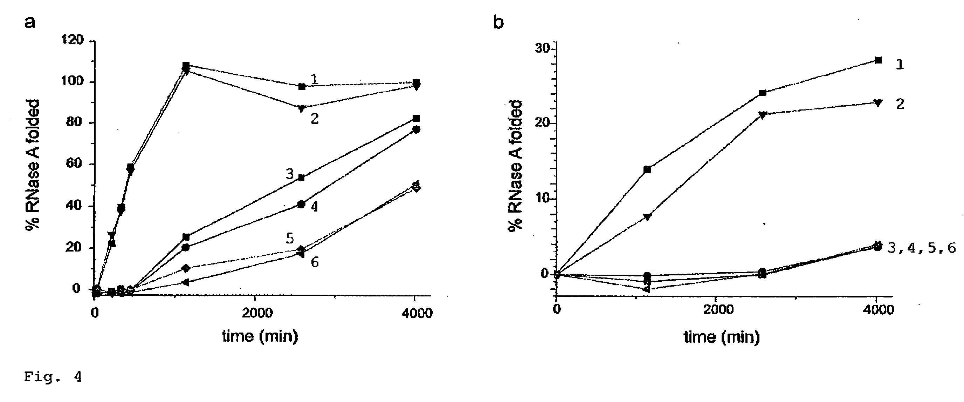

Figure 4 illustrates oxidative folding of 5 µM RNase A by 10 µM cystamine (in blue) or selenocystamine (in red) functionalized CLEAR (triangles), Chemmatrix resin (squares), CLEAR-OX resin (green triangles) and in the presence of no additives (grey diamonds), a) at pH 8.0 and b) at pH 5.0; the curves in Fig. 4a/b are (1) Chemmatrix + selenocystamine, (2) CLEAR + selenocystamine, (3) Chemmatrix + cystamine, (4) CLEAR + cystamine, (5) no additives, and (6) CLEAR-OX;

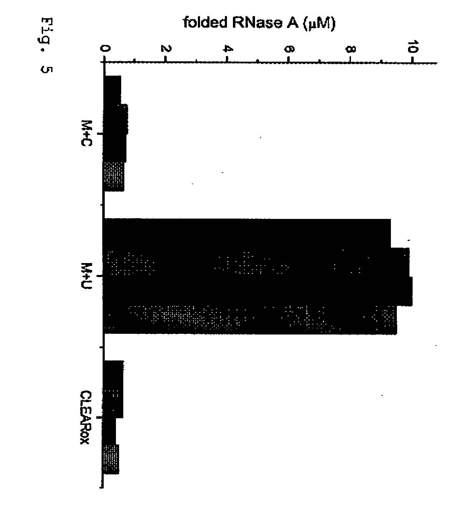

Figure 5 shows the recycling of resin. RNase A (10 µM) was refolded in the presence of 200 µM cystamine or selenocystamine functionalized Chemmatrix resin, or CLEAR-OX. After 2h, the resin and refolded protein were separated by centrifugation and the resin washed prior to another refolding cycle;

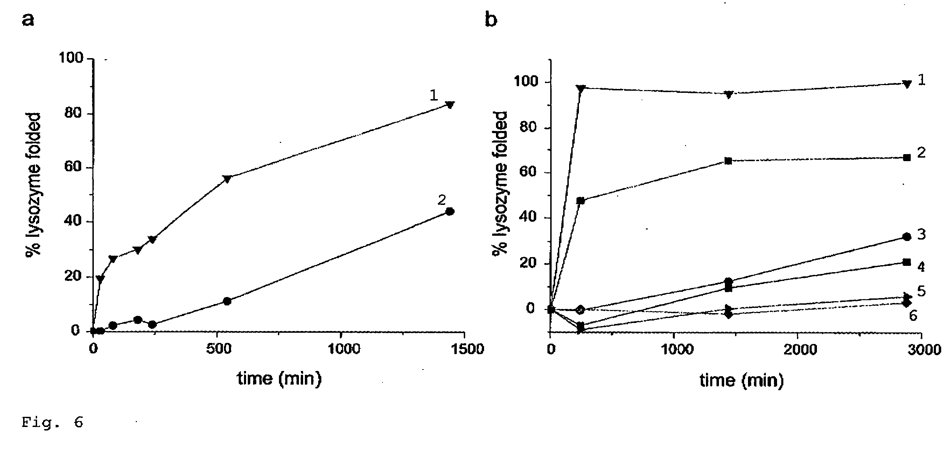

Figure 6 illustrates oxidative folding of lysozyme monitored by an activity assay. a) Oxidative folding of 5 µM lysozyme by 20 µM disulfide (blue circles; 2) and diselenide (red triangles; 1) containing CLEAR resin at pH 8.0 in a buffer containing 2 M urea, b) Oxidative folding of 4.25 mg/ml lysozyme by 200 µM disulfide (C+C in blue circles (3) and M+C in blue squares (4)) or diselenide (C+U in red triangles (1) and M+U in red squares(2)) containing resin, at pH 8.0 in a buffer containing 1.8 M Gdn-HCl. Folding with 200 µM CLEAR-OX and no additives are shown respectively in green (5) and grey (6); and

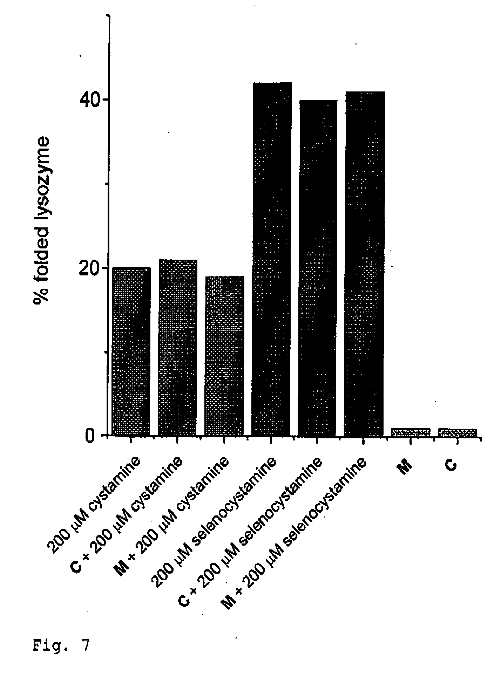

Figure 7 shows the effect of water-compatible CLEAR and Chemmatrix resin on the folding efficiency. Lysozyme (4.5 mg/ml) was folded in a buffer containing 1.8 Gdn-HCl, pH 8.0, in the presence of cystamine or selenocystamine.

Additionally, 4 mg/ml of non-functionalized C (CLEAR) or M (Chemmatrix) were added. The folding efficiency was monitored by a standard activity assay after 24h head-over-head shaking at room temperature.

Resin bound glutathione and selenoglutathione were synthesized in an analogous fashion as previously published (see Figure 1), ²⁹ but here on HMBA functionalized resins, allowing for acidic cleavage of side-chain protecting groups without cleaving the peptide from the resin. To avoid the typical harsh conditions for the deprotection of selenocysteine, the recently published method using 2,2'-dithiobis(5-nitropyridine) in TFA was adopted.³⁰ Since this design relies on the formation of intermolecular disulfide or diselenide bonds between peptides bound to the polymer and, synthesis of amino acid building blocks is necessary, a second approach was devised. Here, resin bound cystamine and selenocystamine were synthesized using a versatile 2,3-dibromopropionyl chloride linker, allowing for intramolecular formation of disulfide or diselenide bonds (Figure 2). After synthesis, the resins were reduced with DTT to remove cystamine/selenocystamine not bound to the polymer and subsequently oxidized once more with K₃Fe(CN)₆. The loading of the resins was determined by Ellman's assay² and a colorimetric assay for disulfide/diselenide bonds (see examples) .³¹ The incorporation of the HMBA linker allowed for basic cleavage of the disulfides/diselenides from the resin and subsequent characterization showed that the desired products are present on the resins. With these water-compatible resins in hand, two proteins were folded under a variety of conditions.

The small enzyme ribonuclease A (RNase A) requires its four disulfide bonds to catalyze the conversion of cCMP to CMP, allowing for the direct coupling between oxidative folding and activity. In a simple experiment 5 µM of RNase A was folded in the presence of 1 mg of various resins (in disregard of resin loading) and showed a clear advantage of all diselenide-containing resins (see Figure 3). Since the cystamine/selenocystamine resins are easier to synthesize, better defined, require only commercially available chemicals and, showed oxidative protein folding behavior as good as the glutathione or selenoglutathione resins, the focus was put on these small molecular disulfide/diselenide resins (Figure 3). Oxidative folding of 5 µM RNase A in the presence of 10 µM resin bound disulfide/diselenide shows the big advantage of diselenide resins over disulfide resins. Where disulfide-containing resins give about 20% yield of native protein, diselenide-containing resins fold RNase A to completion in 24h (Figure 4a). Interestingly, the observed rate is only slightly slower than folding with an optimized 0.2 mM GSSG and 1 mM GSH redox buffer.²⁸ Further, 10 µM CLEAR-OX does not show any advantage over folding in buffer alone. As in solution, RNase A can be folded at pH 5.0 by diselenide containing resins (Figure 4b), whereas disulfide-containing resins (as well as CLEAR-OX) are unable to show any significant amount of folded protein. However, as demonstrated in solution, ²⁸ rate and yield are (severely) hampered at acidic pH.

Major advantages of resin bound reagents are the ease of separation from the reaction mixture and the easy recyclability of the reagent. To show the practicality of resin bound diselenides 10 µM of RNase A was incubated, at pH 8, with 200 µM resin bound selenocystamine, cystamine or 200 µM CLEAR-OX (Figure 5). After 2h, the refolded protein and resin were simply separated by centrifugation. Using diselenide-containing resin RNase A was folded to completion, whereas disulfide-containing resin gave a yield < 10%. The resin was carefully washed and used in multiple refolding cycles and shows good reproducibility and recyclability (Figure 5).

The oxidative folding of lysozyme is hampered by non-productive competition of correct folding with unspecific aggregation. Under optimized redox buffer conditions (0.2 mM GSSG and 1 mM GSH), typically a yield of 60% refolded protein is obtained in 2 M urea, pH 8.^{23, 24} Here, with only 20 µM diselenide-containing resin, lysozyme was folded to completion in 20h (Figure 6a).

Typically, oxidative protein folding is performed at low protein concentrations (1 to 5 µM) to prevent aggregation. However, working with such low concentrations is impractical in biotechnological production of proteins. Lysozyme has been successfully folded at 1 mg/ml (70 µM) , in the presence of denaturants and high concentrations of a redox buffer.³² The power of selenocystamine functionalized resins is further exemplified by the successful folding of 4.25 mg/ml (290 µM) lysozyme in 1.8 M Gdn-HCl (Figure 6b).

The question arises whether these resins have a chaperone effect by itself, like BSA. However, the oxidative folding of lysozyme in the presence of a redox buffer and non-functionalized resin, shows no significant increase in yield (see Figure 7). Also, non-functionalized resins alone are inactive as oxidative protein folding reagents. Interestingly, although efficient at oxidizing cysteines,⁷ the commercially available CLEAR-OX resin is not able to fold RNase A and lysozyme to any appreciable extent. Maybe this is not unexpected since Ellman's reagent (DTNB) is also not used for oxidative protein folding. Additionally, a diselenide-containing Wang resin shows no efficient oxidative folding of RNase A (Figure 3), suggesting that the water compatibility of Chemmatrix and CLEAR resins is a necessity for successful oxidative protein folding. The combination of small molecular diselenides and water compatible resins is the key to productive oxidative protein folding.

The disulfide- and diselenide-containing resins show a similar behavior as the same molecules in solution,^{26,27} but allow for practical oxidative protein folding with easy reagent recycling. Pure polyethylene glycol resins are organic solvent and water compatible, and although not showing any chaperone activity, are essential for the success of the disulfide- and diselenide-containing resins. The application of the here developed oxidative protein folding resins is such that it can be put to standard use in the laboratory like for example Ni-NTA beads. Further, the biotechnological production of proteins can be significantly enhanced and simplified by diselenide-containing resins. Overproduction of proteins in inclusion bodies, followed by denaturation and simple and fast refolding is one of the most efficient methods of protein production, in particular on the industrial large scale level. As demonstrated the combination of small molecular diselenide compounds and solid supported reagents, provides for a far more efficient oxidative protein folding.

### Examples

### General

Aminomethyl Chemmatrix resin was obtained from Fluka. Base CLEAR and CLEAR-OX resins were obtained from Peptides International. All other chemicals were obtained from ABCR, Sigma-Aldrich, Fluka and Axon lab. Boc-Glu(α-OtBu)-OPfP and Fmoc-Sec(Mob)-OPfp were synthesized as previously described²⁹.

### HMBA on Chemmatrix and CLEAR resin

After swelling the aminomethyl terminated resin in DMF, 4 eq. 4-hydroxymethylbenzoic acid, 4 eq. DIPCDI and 4 eq. HOBt in DMF were added and the solution shaken overnight at room temperature. The resin was extensively washed with DMF, CH₂Cl₂ and EtOH, and Kaiser-test³³ showed no free amines.

### Fmoc-Gly-HMBA on Chemmatrix and CLEAR resin

Loading of Fmoc-Gly-OH (4 eq.) on HMBA functionalized resin was performed in the presence of 4 eq. MSNT, 8 eq. NMI and 12 eq. DIPEA in CH₂Cl₂ (2.5h at room temperature). After extensive washing with DMF, CH₂C1₂ and EtOH, and drying, the loading was determined by Fmoc-release assay using 4-methylpiperidine in NMP (1 mmol/g).

### Glutathione-HMBA on Chemmatrix and CLEAR resin

The Fmoc-Gly-HMBA-resins were deprotected with 4-methylpiperidine in NMP (2x for 5 min) and extensively washed prior to addition of 2 eq. Fmoc-Cys(Trt)-OH in the presence of HBTU/HOBt (1:1, 2 eq.), followed by deprotection with 4-methylpiperidine, washing, addition of 2 eq. Boc-Glu(α-OtBu)-OPfP and 1 eq. HOBt in NMP and a final deprotection with a cleavage cocktail containing 10:0.1:0.1:0.1 TFA/TIPS/PhOH/H₂O, followed by extensive washing and drying.

### Selenoglutathione-HMBA on Chemmatrix and CLEAR resin

Fmoc-Gly-HMBA-resins were deprotected with 4-methylpiperidine in NMP (2x for 5 min) and extensively washed prior to addition of 2 eq. Fmoc-Sec(Mob)-OPfp in the presence of HOBt (1 eq.), followed by deprotection with 4-methylpiperidine, washing, addition of 2 eq. Boc-Glu(α-OtBu)-OPfP and 1 eq. HOBt in NMP and a final deprotection with a cleavage cocktail containing 10:0.1:0.1:0.1:1.3 eq. TFA/TIPS/PhOH/H₂O/DTNP, followed by extensive washing and drying³⁰.

### Cystamine and selenocystamine on Chemmatrix and CLEAR resin

To HMBA functionalized resin, 4 eq. 2,3-dibromopropionyl chloride and 4 eq. DIPEA, in DMF, were added and shaken for 4h at rT. After extensive washing, 4 eq. of cystamine or selenocystamine and 4 eq. of DIPEA in DMF were added and shaken overnight at room temperature. After extensive washing, the resin was incubated for 1h with 1 M DTT in 100 mM Tris-HCl, 2 mM EDTA, pH 8.0. After extensive washing, the resin was subsequently incubated for 1h with 0.5 M K₃Fe(CN)₆ prior to extensive washing with H₂O, EtOH and drying.

### Characterization of resins

Both the thiol-responsive Ellman's assay² and a disulfide-sensitive colorimetric assay were performed on the resins²⁹. Ellman's assay showed no free selenols on diselenide-containing resins, however cystamine containing resins could be fully reduced by 5,5'-dithiobis(2-nitrobenzoic acid) and showed a similar loading as determined by a disulfide-sensitive colorimetric assay. Resin loading (mmol/g): CLEAR-OX: 0.17, C-GSSG: 0.23, C-GSeSeG:0.20, C+C: 0.04, C+U: 0.02, M+GSSG: 0.22, M+GSeSeG: 0.20, M+C: 0.09, M+U: 0.04. The incorporation of the base labile HMBA linker allows for facile cleavage of the molecules of the resin. Cleavage with a mixture of Et₃N, THF and propylamine (5:5:1) overnight at 50 °C showed by LCMS (ESI-MS) that the desired molecules were present on the resins. ESI-MS. GSSG functionalized resin cleaved with NaOH: calcd. (M+H) 613.1 found 613.0, GSeSeG functionalized resin cleaved with NaOH: calcd. (M+H) 709.0 found 709.1, cystamine functionalized resins cleaved with NaOH (1,2,5,8-dithiadiazecane-6-carboxylic acid): calcd. (M+H) 222.1 found 222.2, selenocystamine (1,2,5,8-diselenadiazecane-6-carboxylic acid): calcd. (M+H) 317.9 found 318.0, cystamine functionalized resins cleaved with propylamine (N-propyl-1,2,5,8-dithiadiazecane-6-carboxamide): calcd. (M+H) 263.1 found 263.5 and selenocystamine functionalized resins cleaved with propylamine (N-propyl-1,2,5,8-diselenadiazecane-6-carboxamide): calcd. (M+H) 359.0 found 359.3.

### Oxidative folding of ribonuclease A

RNase A (500 µM) was reduced with 100 mM DTT in 6 M Gdn-HCl, 100 mM Tris-HCl, 2 mM EDTA, pH 8.0 overnight at room temperature and purified by preparative HPLC. The lyophilized powder was dissolved in 10 mM HCl at a concentration of 500 µM and diluted in 100 mM Tris-HCl, 2 mM EDTA, pH 8.0 or in 2 mM EDTA, 100 mM acetate buffer (pH 5.0) and split into different reaction mixtures, to which various amounts of (dissolved) resins were added, prior to incubation at rT under orbital shaking. Aliquots were removed at certain times and quenched with 1 M HCl. The aliquots were assayed for active RNase A by its ability to catalyze the hydrolysis of cCMP, spectrophotometrically monitored at 296 nm ³⁸.

### Resin recycling

A stock solution of reduced and denatured RNase A in 10 mM HCl (500 µM) was diluted 50x into 100 mM Tris-HCl, 2 mM EDTA, pH 8.0, and split into different refolding mixtures, to which various amounts of (dissolved) resins were added (200 µM). The reaction mixtures were shaken for 2h at room temperature, spun down, supernatant removed and quenched with 1 M HCl and the resin washed 4x with 100 mM Tris-HCl, 2 mM EDTA, pH 8.0 buffer and EtOH (cycle 1). To the resin another aliquot of freshly prepared refolding mixture was added and this cycle repeated four times.

### Oxidative folding of lysozyme

Hen egg white lysozyme (50 mg/ml) was reduced with 1 M DTT in 6 M Gdn-HCl, 100 mM Tris-HCl, 2 mM EDTA, pH 8.0 overnight at room temperature and purified by preparative HPLC. The lyophilized powder was dissolved at 5 mg/ml in 10 mM HCl, and diluted to a concentration of 5 µM into 2 M urea, 100 mM Tris-HCl, 2 mM EDTA, pH 8.0, and split into different reaction mixtures, to which various amount of (dissolved) resins were added, prior to incubation at rT under orbital shaking. Aliquots were removed at certain times and quenched with 1 M HCl. The aliquots were assayed for lysozyme activity by a standard *Micrococcus Lysodeikticus* cell lysis assay³⁹.

In a separate experiment, reduced and lyophilized lysozyme was dissolved at approximately 500 mg/ml in 6 M Gdn-HCl, 100 mM Tris-HCl, 2 mM EDTA, pH 5.0. The protein was diluted 100x into 1.8 M Gdn-HCl, 100 mM Tris-HCl, 2 mM EDTA, pH 8.0, and added to different reaction vessels containing various amounts of (dissolved) resin, prior to incubation at rT under head-over-head shaking. Aliquots were removed at certain times, quenched with 1 M HCl and assayed for lysozyme activity.

The chaperone effect of water-compatible CLEAR and Chemmatrix resin was studied by the folding of lysozyme (4.5 mg/ml) in a buffer containing 1.8 Gdn-HCl, 100 mM Tris-HCl, 2 mM EDTA, pH 8.0, in the presence of cystamine or selenocystamine and non-functionalized resins (4 mg/ml). The folding efficiency was measured after 24h head-over-head shaking at room temperature, using the activity assay described previously.

### References

1. Clark, B. R., and Pai, M. (1996) Polymeric resin for disulfide bond synthesis, in Patent cooperation treaty application*.*
2. Ellman, G. L. (1959) Tissue sulfhydryl groups, Arch. Biochem. Biophys. 82, 70-77.
3. Berezhkovskiy, L., Pham, S., Reich, E. P., and Deshpande, S. (1999) Synthesis and kinetics of cyclization of MHC class II-derived cyclic peptide vaccine for diabetes, J. Pept. Res. 54, 112-119.
4. Lipp, M. M., Lee, K. Y. C., Zasadzinski, J. A., and Waring, A. J. (1996) Phase and morphology changes in lipid monolayers induced by SP-B protein and its amino-terminal peptide, Science 273, 1196-1199.
5. Annis, I., Chen, L., and Barany, G. (1998) Novel solid-phase reagents for facile formation of intramolecular disulfide bridges in peptides under mild conditions, J. Am. Chem. Soc. 120, 7226-7238.
6. Darlak, K., Wiegandt Long, D., Czerwinski, A., Darlak, M., Valenzuela, F., Spatola, A. F., and Barany, G. (2004) Facile preparation of disulfide-bridged peptides using the polymer-supported oxidant CLEAR-OX, J. Pept. Res. 63, 303-312.
7. Green, B. R., and Bulaj, G. (2006) Oxidative folding of conotoxins in immobilized systems, Prot. Pept. Lett. 13, 67-70.
8. Cleland, W. W. (1964) Dithiothreitol, a new protective reagent for SH groups, Biochemistry 3, 480-482.
9. Shimizu, H., Fujimoto, K., and Kawaguchi, H. (1999) Refolding of protein using thiol-carrying latex particles, Colloid. Surface A 153, 421-427.
10. Shimizu, H., Fujimoto, K., and Kawaguchi, H. (2000) Improved refolding of denatured/reduced lysozyme using disulfide-carrying polymeric microspheres, Colloid. Surface B 18, 137-144.
11. Woycechowsky, K. J., Hook, B. A., and Raines, R. T. (2003) Catalysis of protein folding by an immobilized small-molecule dithiol, Biotechnol. Prog. 19, 1307-1314.
12. Rapp, W. (1996) PEG grafted polystyrene tentacle polymers: physico-chemical properties and application in chemical synthesis, Weinheim VCH.
13. Rademann, J., Grøtli, M., Meldal, M., and Bock, K. (1999) SPOCC: A resin for solid-phase organic chemistry and enzymatic reactions on solid phase, J. Am. Chem. Soc. 121, 5459-5466.
14. Kempe, M., and Barany, G. (1996) CLEAR: A novel family of highly cross-linked polymeric supports for solid-phase peptide synthesis, J. Am. Chem. Soc. 118, 7083-7093.
15. Garcia-Martin, F., Quintanar-Audelo, M., Garcia-Ramos, Y., Cruz, L. J., Gravel, C., Furic, R., Cote, S., Tulla-Puche, J., and Albericio, F. (2006) ChemMatrix, a poly(ethylene glycol)-based support for the solid-phase synthesis of complex peptides, J. Comb. Chem. 8, 213-220.
16. Johnson, E. C. B., Durek, T., and Kent, S. B. H. (2006) Total chemical synthesis, folding, and assay of a small protein on a water-compatible solid support, Angew. Chem., Int. Ed. Engl. 45, 3283-3287.
17. Frutos, S., Tulla-Puche, J., Albericio, F., and Giralt, E. (2007) Chemical synthesis of 19F-labeled HIV-1 protease using Fmoc-chemistry and chemmatrix resin, Int. J. Pept. Res. Ther. 13, 221-227.
18. Garcia Martin, F., and Albericio, F. (2008) Solid supports for the synthesis of peptides. From the first resin used to the most sophisticated in the market, Chimica Oggi 26, 29-34.
19. Hochuli, E., Döbeli, H., and Schacher, A. (1987) New metal chelate adsorbent selective for proteins and peptides containing neighbouring histidine residues, J. Chromatogr. A 411, 177-184.
20. Ley, S. V., Baxendale, I. R., Brusotti, G., Caldarelli, M., Massi, A., and Nesi, M. (2002) Solid-supported reagents for multi-step organic synthesis: Preparation and application, Farmaco 57, 321-330.
21. Fink, A. L. (1999) Chaperone-mediated protein folding, Physiol. Rev. 79, 425-449.
22. Puig, A., and Gilbert, H. F. (1994) Protein disulfide isomerase exhibits chaperone and anti-chaperone activity in the oxidative refolding of lysozyme, J. Biol. Chem. 269, 7764-7771.
23. Van Den Berg, B., Chung, E. W., Robinson, C. V., and Dobson, C. M. (1999) Characterisation of the dominant oxidative folding intermediate of hen lysozyme, J. Mol. Biol. 290, 781-796.
24. Van Den Berg, B., Chung, E. W., Robinson, C. V., Mateo, P. L., and Dobson, C. M. (1999) The oxidative refolding of hen lysozyme and its catalysis by protein disulfide isomerase, EMBO J. 18, 4794-4803.
25. Zhou, B. R., Liang, Y., Du, F., Zhou, Z., and Chen, J. (2004) Mixed macromolecular crowding accelerates the oxidative refolding of reduced, denatured lysozyme: Implications for protein folding in intracellular environments, J. Biol. Chem. 279, 55109-55116.
26. Van Den Berg, B., Wain, R., Dobson, C. M., and Ellis, R. J. (2000) Macromolecular crowding perturbs protein refolding kinetics: Implications for folding inside the cell, EMBO J. 19, 3870-3875.
27. Buczek, O., Green, B. R., and Bulaj, G. (2007) Albumin is a redox-active crowding agent that promotes oxidative folding of cysteine-rich peptides, Biopolymers 88, 8-19.
28. Beld, J., Woycechowsky, K. J., and Hilvert, D. (2008) Catalysis of oxidative protein folding by small-molecule diselenides, Biochemistry 47, 6985-6987.
29. Beld, J., Woycechowsky, K. J., and Hilvert, D. (2007) Selenoglutathione: Efficient oxidative protein folding by a diselenide, Biochemistry 46, 5382-5390.
30. Harris, K. M., Flemer Jr, S., and Hondal, R. J. (2007) Studies on deprotection of cysteine and selenocysteine side-chain protecting groups, J. Pept. Sci. 13, 81-93.
31. Thannhauser, T. W., Konishi, Y., and Scheraga, H. A. (1984) Sensitive quantitative analysis of disulfide bonds in polypeptides and proteins, Anal. Biochem. 138, 181-188.
32. Dong, X. Y., Shi, J. H., and Sun, Y. (2002) Cooperative effect of artificial chaperones and guanidinium chloride on lysozyme renaturation at high concentrations, Biotechnol. Prog. 18, 663-665.
33. Kaiser, E., Colescott, R. L., Bossinger, C. D., and Cook, P. I. (1970) Color test for detection of free terminal amino groups in the solid-phase synthesis of peptides, Anal. Biochem. 34, 595-598.
34. Milton, M. D., Khan, S., Singh, J. D., Mishra, V., and Khandelwal, B. L. (2005) A facile access to chalcogen and dichalcogen bearing dialkylamines and diols, Tetrahedron Lett. 46, 755-758.
35. Klayman, D. L., and Griffin, T. S. (1973) Reaction of selenium with sodium-borohydride in protic solvents - facile method for introduction of selenium in organic molecules, J. Am. Chem. Soc. 95, 197-200.
36. Yang, X., Wang, Q., Tao, Y., and Xu, H. (2002) A modified method to prepare diselenides by the reaction of selenium with sodium borohydride, J. Chem. Res., part S, 160-161.
37. Ścianowski, J. (2005) Convenient route to dialkyl diselenides from alkyl tosylates. Synthesis of di(cis-myrtanyl) diselenide, Tetrahedron Lett. 46, 3331-3334.
38. Lyles, M. M., and Gilbert, H. F. (1991) Catalysis of the oxidative folding of ribonuclease A by protein disulfide isomerase: Dependence of the rate on the composition of the redox buffer, Biochemistry 30, 613-619.
39. Shugar, D. (1952) The measurement of lysozyme activity and the ultra-violet inactivation of lysozyme, Biochim. Biophys. Acta 8, 302-309.

## Claims

1. A resin comprising a water-compatible polymer and a diselenide compound wherein the diselenide compound is either directly or by a linker covalently bound to the polymer.

2. Resin according to claim 1, wherein the water-compatible polymer is selected from the group consisting of poly(alkylene oxide) (PEO), poly (ethylene glycol) (PEG), proteins, polysaccharides, poly(vinyl alcohol) (PVA), polyacrylat (PA), polymethyloxazoline (PMOXA), polyethyloxazoline (PEOXA), polyvinylpyrrolidone (PVP), polylysine, polyarginine, polytyrosine, polyglutamine, polyglutamic acid, polyasparigine, polyaspartic acid, polyserine, polythreonine and polyhistidine, polyallylamine, polyethyleneimine, and any mixture thereof.

3. Resin according to claim 1 or 2, wherein the diselenide compound is bound to the polymer by a linker.

4. Resin according to any of claims 1 to 3, wherein the linker is selected from the group consisting of alkyl chains with 1 to 20 carbon atoms, which may be straight or branched and saturated or unsaturated, dimethylbenzene, and 4-hydroxymethyl benzoic acid.

5. Resin according to any of claims 1 to 4, wherein the linker is 4-hydroxymethyl benzoic acid.

6. Resin according to any of claims 1 to 5, wherein the diselenide compound has a molecular mass of less than 5000Da, preferably less than 2500Da, more preferably less than 1000Da, even more preferably less than 500Da and most preferably of less than 300Da.

7. Resin according to claim 6, wherein the diselenide compound is selected from the group consisting of either straight chain or branched alkyl diselenide compounds, aromatic diselenide compounds.

8. Resin according to claim 6, wherein the diselenide compound is selected from the group consisting of selenocystine, selenogluthatione, selenocystamine, bis(amino)diphenyldiselenid and 5,5'-diselenobis-(2-nitrobenzoic acid).

9. Method for the production of a diselenide-resin according to any of claims 1 to 8, comprising the steps,
a) optionally coupling a linker to a resin;
b) coupling a diselenide compound to the resin; and
c) treating the resin with a reducing reagent, followed by treating the resin with an oxidizing reagent.

10. Method according to claim 9, wherein the resin is a polyethylene glycol based resin functionalized with aminomethyl groups.

11. Method for the production of a resin according to claim 10, comprising the steps
a) coupling of 4-hydroxymethyl benzoic acid to the resin, followed by subsequent coupling of dibromopropionyl chloride;
b) coupling of selenocystamine to the resin; and
c) treating the resin with the reducing reagent dithiothreitol followed by treating the resin with the oxidizing reagent K₃Fe (CN) ₆.

12. Use of resin according to any of claims 1 to 8 for oxidative protein folding.
